# EUROPEAN PATENT APPLICATION

(11) **EP 1 314 415 A1**
(43) Date of publication of application: **28.05.2003**
(21) Application number: 01947978.1
(22) Date of filing: 11.07.2001
(51) Int. Cl.: A61K 7/00, A61K 7/48, A61K 7/02, A61K 7/06, A61K 7/32, A61K 7/40

(54) **COSMETICS**

(30) Priority: 12.07.2000 JP 2000211319
(71) Applicant: Shin-Etsu Chemical Co., Ltd., Chiyoda-ku, Tokyo 100-0004 (JP)
(72) Inventor: ICHINOHE, Shoji, Silicone Elect. MaterialCenter, matsuidacho, Usuigun, Gunma 379-0224 (JP); SHIMIZU, Toru Shin-Etsu Chemical Co., Ltd., Tokyo 100-0004 (JP)
(74) Representative: Bubb, Antony John Allen
(86) International application number: JP0106026
(87) International publication number: WO02003928

(57) **Abstract**

The present invention is a cosmetic material characterized by comprising silicone-modified wax wherein low-molecular-weight polyethylene and/or low-molecular-weight polypropylene is linked to silicone via ester linkage.

The present cosmetic material spreads easily and gives a refreshing feel to users. In addition, it has strong repellency to sweat and water, but does not impair moderate transpiration of moisture when it is coated. And the coating thereof imparts elasticity, smoothness, emollient effect and so on. Further, it is excellent in natural luster-imparting effect and storage stability.

## Description

### Technical Field

The present invention relates to a cosmetic material using silicone-modified wax, specifically carboxyl group-and/or carboxylic acid anhydride-containing polyethylene wax and/or polypropylene wax modified with a silicone. More specifically, the invention is concerned with a cosmetic material in which the wax specified above is incorporated to impart thereto a good spread capability, high possibility of giving a feeling of refreshment to users and strong repellencies to sweat and water. When applied to skin, the present cosmetic material can therefore ensure elasticity, smoothness, emollient effect, natural luster and good keeping qualities.

### Background of the Invention

Hitherto, cosmetic materials containing liquid unctuous agents, such as paraffin, ester, higher alcohol, glyceride and the like, have been used for the purpose of adding elasticity, smoothness, emollient effect and so on. However, these cosmetic materials have drawbacks of producing a greasy feeling, a tacky feeling and a feeling of being covered with an oil film. So it has also been known to mix silicone oils, such as dimethylpolysiloxane, in the cosmetic materials of the aforementioned compositions with the intention of reducing those greasy, tacky and filmy feels.

Although silicone oils have good spread capability, high smoothness and strong repellency, they have a disadvantage of being inferior in compatibility with liquid oils of hydrocarbon type. In addition, silicone oils have low surface tension, so they spread quickly and have short residence time. Such being the case, attempts to solidify silicone oils have been made.

As materials capable of providing silicone oils in a solid state, the compound having a silicone chain to which alkyl groups are introduced (Japanese Patent Laid-Open (hereinafter referred to as "Tokkai") Hei 2-64115), the compound having a silicone chain to which 21-30C aliphatic alcohol or acid ester groups are introduced (Tokkai Hei 10-500431), the acrylate silicone (Tokkai Hei 2-132141) and so on have been reported. With the compounds having silicone chains to which alkyl groups and long chain fatty acid ester groups are introduced respectively, however, it is hard to smoothly solidify silicone oils themselves. In general, therefore, solidification of those compounds are made in the presence of liquid oil, such as ester oil, triglyceride oil, paraffin oil or so on, because their affinities for waxes are enhanced under such a condition. In the case of the acrylate silicone, on the other hand, the silicone oil by itself can form a solidified matter. However, this solidified matter is resinous and lacking in smoothness suitable for cosmetics. Under these circumstances, solid substances capable of bringing silicone oil characteristics into full play, and that with highly satisfactory smoothness, still haven't been found.

As a result of our intensive studies of solidified silicone oils suitable for cosmetic materials, it has been found that silicone oil can be made into a smooth solid by mixing with silicone-modified wax containing an ester linkage in a molecule which is produced by reacting carboxyl group- and/or carboxylic anhydride-containing polyethylene and/or polypropylene wax with a silicone compound, preferably a silicone compound containing only one epoxy group in a molecule or a silicone compound containing both only one carbon-carbon double bond and only one epoxy group in a molecule, and then subjecting the resultant reaction product to hydrosilylation reaction with a silicone compound, preferably a silicone compound containing only one silicon-hydrogen bond in a molecule, thereby achieving the invention.

Therefore, an object of the present invention is to provide a cosmetic material having excellent usability and high stability over a long term, specifically a cosmetic material which can be spread lightly, easily and smoothly without tackiness when applied to skin and arouse dry, smooth and refreshing feelings in users, and further has not only strong repellency to sweat and water but also properties of imparting elasticity, smoothness, emollient effect and natural luster to skin without impairing skin's moderate transpiration by application thereof.

### Disclosure of the Invention

The present invention is a cosmetic material containing silicone-modified wax as Component (A), characterized in that the silicone-modified wax is wax in which low-molecular-weight polyethylene and/or low-molecular-weight polypropylene is linked to a silicone via an ester linkage.

The present cosmetic material can be spread lightly and produce a feeling of refreshment when applied to skin. More specifically, it can be spread easily and smoothly without tackiness when applied to skin and arouse dry, smooth and refreshing feelings in users. Further, the present cosmetic material has good long-term stability as well as excellent usability since it has not only strong repellency to sweat and water but also properties of imparting elasticity, smoothness, emollient effect and natural luster to skin without impairing skin's moderate transpiration by application thereof.

### Best Mode for Carrying out the Invention

It is appropriate that the low-molecular-weight, carboxyl group- and/or carboxylic anhydride-containing polyethylene and/or polypropylene used as a starting material of the present silicone-modified wax has a molecular weight of 1,000 to 50,000. In particular, maleic anhydride-grafted polyethylene and polypropylene having an acid value of 10 to 100 KOH mg/g are advantageously used as such a starting material. Although these polyethylene and polypropylene are marketed by, e.g., Mitsui Chemicals and Sanyo Chemical Industries, Ltd. under the trade names of Mitsui Hiwax and Yumex, respectively, there have been no cases of using such high-melting-point silicone wax in cosmetic materials. However, the present invention enables smooth solidification of silicone oils themselves by taking advantage of their high-melting-point characteristics.

Methods for synthesizing the aforesaid silicone-modified wax used in the invention fall into two broad categories. The methods in the first category involve reaction of carboxyl group- and/or carboxylic anhydride-containing polyethylene (or polypropylene) wax with an epoxy group-containing dimethylsilicone, preferably a dimethylsilicone having an epoxy group at only one end of its chain. Therein, a silicone compound having two or more epoxy groups at both ends or/and in the interior of its molecule may be also used so far as it causes gelling in the reaction product. In carrying out the reaction, solvents may be used or not, and it is appropriate that the reaction temperature be adjusted to 100°C or higher, especially 140°C or higher.

When no solvent is used in the reaction, it is preferable to adjust the reaction temperature so as to be higher than the melting point of the wax used. The reaction may be conducted in the absence or presence of a catalyst. In the case of using a catalyst, tertiary amines, metal soaps, imidazole compounds and so on are suitable as the catalyst. As to the proportion of the silicone compound to the wax in the reaction, it is appropriate that the silicone compound be used in an amount of 0.2 to 5 equivalents, particularly 0.5 to 2 equivalents, in terms of epoxy group when the wax is used in an amount of 1 equivalent in terms of carboxyl group. In respect of reactivity, cyclic epoxy groups are preferred to linear epoxy groups. Although the reaction time depends on the reaction temperature, it usually takes 5 to 10 hours to complete the reaction.

The methods in the second category involve, as the first step, reaction of a compound containing an epoxy group and a double bond in its molecule, e.g., Celoxide 2,000 (trade name of vinylcyclohexene epoxide produced by DaiCel Chemical Industries, Ltd.), with carboxyl group and/or carboxylic anhydride-containing polyethylene (or polypropylene) wax. Since the compound, such as Celoxide 2,000, can be removed by heating under reduced pressure after the reaction, it is advantageous from the viewpoint of enhancing reaction efficiency to use 1.2 to 5 equivalents of Celoxide 2,000 or the like with respect to 1 equivalent of carboxyl group in the wax. The suitable reaction conditions are the same as in the former case of using an epoxysilicone compound.

By distilling off Celoxide 2,000 or the like through heating under reduced pressure, double bond-introduced polyethylene (or polypropylene) wax can be obtained. It is appropriate that addition reaction between this wax and a Si-H bond-containing silicone be carried out at the melting temperature of the wax or higher when conducted in the absence of a solvent. When the addition reaction is conducted in the presence of a solvent, on the other hand, the suitable reaction temperature is in the range of to 140°C. As a catalyst for the addition reaction, precious metal catalysts, such as chloroplatinic acid, can be used. Therein, it is appropriate that the silicone compound be used in an amount of 0.1 to 5 equivalnets, particularly 0.2 to 1 equivalent, in terms of Si-H group when the wax is present in an amount of one equivalent in terms of double bond. In the case of using a solvent, the intended silicone-modified wax can be obtained by distilling off the solvent through heating under reduced pressure.

The present silicone compound can be used for a wide variety of purposes. Specifically, it is highly suitable as an ingredient in every kind of cosmetic material externally applied to skin or hair, including skin-care cosmetics, makeup cosmetics, hair-care cosmetics, antiperspirant cosmetics, UV protective cosmetics and so on. In such cosmetics, it is possible to mix the silicone-modified wax of Component (A) in a proportion of 0.1 to 95 weight % to total cosmetic material. In a special case of solid-state or stick-shaped cosmetic materials wherein powdery ingredients account for at most 25 % of total formulation, the suitable proportion of the present silicone-modified wax compounded is from 5 to 95 weight % to total cosmetic material. In another case of solid cosmetics wherein powdery ingredients are contained in a proportion of at least 80 weight % to total formulation, it is appropriate to mix the present silicone-modified wax in a proportion of 0.1 to 10 weight % to total cosmetic material.

The present cosmetic materials can further contain as Component (B) one or more kinds of unctuous agents depending on the desired purposes thereof. The unctuous agents used together may be in any of solid, semisolid and liquid states, provided that they have so far been used for general cosmetics.

More specifically, not only natural animal and vegetable fats and oils but also semi-synthetic fats and oils can be mixed as unctuous agents in the present cosmetic materials, with examples including avocado oil, linseed oil, almond oil, Chinese wax, perilla oil, olive oil, cacao butter, kapok wax, kaya oil, carnauba wax, liver oil, candellila wax, beef tallow, beef foot oil, beef bone fat, hydrogenated beef tallow, apricot kernel oil, spermaceti, hydrogenated oil, wheat germ oil, sesame oil, rice germ oil, rice bran oil, sugar cane wax, sasanqua oil, safflower oil, shea butter, Chinese tung oil, cinnamon oil, jojoba wax, shellac wax, turtle oil, soybean oil, tea seed oil, tsubaki oil, evening primrose oil, corn oil, lard, rape seed oil, Japanese tung oil, rice-bran wax, germ oil, horse fat, persic oil, palm oil, palm kernel oil, castor oil, hydrogenated castor oil, methyl ester of caster oil fatty acid, sunflower oil, grape seed oil, bayberry wax, jojoba oil, macadamia nut oil, beeds wax, mink oil, cottonseed oil, cotton wax, Japan wax, haze kernel oil, montan wax, coconut oil, hydrogenated coconut oil, tricoconut oil fatty acid glyceride, mutton-tallow, peanut oil, lanolin, liquid lanolin, reduced lanolin, lanolin alcohol, hard lanolin, lanolin acetate, lanolin fatty acid isopropyl, hexyl laurate, POE lanolin alcohol ether, POE lanolin alcohol acetate, polyethylene glycol lanolin fatty acid, POE hydrogenated lanolin alcohol ether, egg yolk oil, and so on. Herein, POE stands for polyoxyethylene.

Examples of hydrocarbon oil which can be mixed include ozokerite, squalane, squalene, ceresine, paraffin, paraffin wax, liquid paraffin, pristane, polyisobutylene, microcrystalline wax, Vaseline and so on; and examples of a higher fatty acid which can be mixed include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, 12-hydroxystearic acid and so on.

Examples of higher alcohol which can also be mixed include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, phytosterol, POE cholesterol ether, monostearyl glycerin ether (batyl alcohol), monooleyl glyceryl ether (cerakyl alcohol) and so on.

Examples of ester oil which can also be mixed include diisobutyl adipate, 2-hexyldecyl adipate, di-2-heptylundecyl adipate, N-alkylglycol monoisostearates, isocetyl isostearate, trimethylolpropane triisostearic acid ester, ethylene glycol di-2-ethylhexanoic acid ester, cetyl 2-ethylhexanoate, trimethylolpropane tri-2-ethylhexanoic acid ester, pentaerythritol tetra-2-ethylhexanoic acid ester, cetyl octanoate, octyldodecyl gum ester, oleyl oleate, octyldodecyl oleate, decyl oleate, neopentyl glycol dicapric acid ester, triethyl citrate, 2-ethylhexyl cinnamate, amyl acetate, ethyl acetate, butyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutaminic acid ester and diisostearyl malic acid; and examples of glyceride oil which can also be mixed include acetoglyceride, triisooctanoic acid glycride, triisostearic acid glyceride, triisopalmitic acid glyceride, monostearic acid glyceride, di-2-heptylundecanoic acid glyceride, trimyristic acid glyceride, myristic acid isostearic acid diglyceride and so on.

As examples of silicone oils which can be mixed as an unctuous agent, mention may be made of organopolysiloxanes ranging from low to high values in viscosity, such as dimethylpolysiloxane, methylphenylpolysiloxane, methylhydrogenpolysiloxane and dimethylsiloxane-methylphenylsiloxane copolymer; cyclic siloxanes, such as octamethylcyclotetrasiloxane, decamethylcyclopentasiloxane, dodecamethylcyclohexasiloxane, tetramethyltetrahydrogen-cyclotetrasiloxane and tetramethyltetraphenylcyclotetra-siloxane; silicone rubbers, such as gummy dimethylpolysiloxanes having high polymerization degrees and gummy dimethylsiloxane-methylphenylsiloxane copolymers having high polymerization degrees; and cyclosiloxane solutions of silicone rubber, trimethylsiloxysilicate, cyclosiloxane solutions of trimethylsiloxysilicate, higer alkoxy-modified silicones such as stearoxysilicone, higher fatty acid-modified silicones, alkyl-modified silicones, amino-modified silicones, fluorine-modified silicones, silicone resin solutions, and the like. Examples of a fluorine-contained unctuous agent which can also be mixed include perfluoropolyether, perfluoro-decaline, perfluorooctaine and the like.

It is appropriate that those unctuous agents mixed as Component (B) account for 1 to 98 weight % of total cosmetic material. Especially when the cosmetic material has a solid form, e.g., a cake or a stick, it is desirable to mix them in a proportion of 5 to 95 weight % to the total cosmetic material.

Furthermore, the present cosmetic materials can contain water as Component (C) according to their individual usage. The suitable proportion of water in total cosmetic material is from 1 to 95 % by weight.

In the present cosmetic materials, one or more of a compound having at least one alcoholic hydroxyl group in its molecular structure can be used as Component (D) depending on their respective purposes.

Examples of a compound having an alcoholic hydroxyl group which can be added include lower alcohol, such as ethanol and isopropanol; sugar alcohol, such as sorbitol and maltose; sterols, such as cholesterol, sitosterol, phytosterol and lanosterol; polyhydric alcohol, such as butylene glycol, propylene glycol and dibutylene glycol; and the like. The suitable proportion of such a compound in total cosmetic material is from 0.1 to 98 % by weight.

In the present cosmetic materials, one or more of a macromolecular compound dissolving or swelling in water can be mixed as Component (E) depending on their individual purposes.

Examples of such a compound include vegetable polymers, such as gum arabic, tragacanth, galactan, carob gum, guar gum, karaya gum, carrageenan, pectin, agar, quince seed, starch (rice, corn, potato, wheat), alge colloid, tranto gum and locust bean gum; microbial polymers, such as xanthan gum, dextran, succinoglucan and pullulan; animal polymers, such as collagen, casein, albumin and gelatin; starch polymers, such as carboxymethyl starch and methylhydroxypropyl starch; cellulose polymers, such as methyl cellulose, ethyl cellulose, methylhydroxypropyl cellulose, carboxymethyl cellulose, hydroxymethyl cellulose, hydroxypropyl cellulose, nitrocellulose, sodium cellulose sulfate, sodium carboxymethylcellulose, crystalline cellulose and cellulose; alginic acid polymers, such as sodium alginate and propylene glycol ester of alginic acid; vinyl polymers, such as polyvinyl methyl ether and carboxyvinyl polymer; polyoxyethylene polymers; polyoxyethylene-polyoxypropylene copolymers; acrylic polymers, such as sodium polyacrylate, polyethyl acrylate and polyacrylamide; other synthetic water-soluble polymers, such as polyethyleneimines and cationic polymers; inorganic water-soluble polymers, such as bentonite, aluminum magnesium silicate, montmorillonite, beidellite, nontronite, saponite, hectorite and silicic acid anhydride; and so on.

In these water-soluble polymers, film-forming agents, such as polyvinyl alcohol and polyvinyl pyrrolidine, are also included. The suitable proportion of polymers as recited above in total cosmetic material is from 0.1 to 25 % by weight.

In addition, the present cosmetic material can contain one or more kinds of powders and/or coloring agents as Component (F) according to the desired purposes.

Any powders can be mixed regardless of shape (whether they are spherical, acicular or tabular), size (whether their sizes are on the order of fume, fine grain or pigment) and structure (whether they are porous or nonporous) as long as they have hitherto been used in conventional cosmetic materials. As examples of such powders and pigments, mention may be made of inorganic powders, organic powders, surfactant metal salt powders, colored pigments, pearl pigments, metallic powder pigments, natural colors and so on.

Examples of an inorganic powder usable herein include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, ruby mica, biotite, lipidolite, silicic acid, silicic anhydride, aluminum silicate, magnesium silicate, aluminum magnesium silicate, calcium silicate, barium silicate, strontium silicate, metal salts of tungstic acid, hydroxyapatite, vermiculite, haidilite, bentonite, montmorillonite, hectorite, zeolite, ceramics powder, calcium secondary phosphate, alumina, aluminum hydroxide, boron nitride, boron nitride, silica and so on.

Examples of an organic powder usable herein include polyamide powder, polyester powder, polyethylene powder, polypropylene powder, polystyrene powder, polyurethane powder, benzoguanamine powder, polymethylbenzoguanamine powder, poly(tetrafluoroethylene) powder, polymethyl methacrylate powder, cellulose powder, silk powder, nylon powder such as 12-nylon powder or 6-nylon powder, fine powders of cross-linked silicones having structures formed by cross-linking dimethylsilicones, fine powders of polymethylsilsesquioxanes, fine powders of resins, such as styrene-acrylic acid copolymer, divinylbenzene-styrene copolymer, vinyl resin, urea resin, phenol resin, fluororesin, silicone, acrylic resin, melamine resin, epoxy resin and polycarbonate resin, microcrystalline fiber powder, starch powder, lauroyl lysine powder and so on.

Examples of a surfactant metal salt powder (metal soap powder) usable herein include powders of zinc stearate, aluminum stearate, calcium stearate, magnesium stearate, zinc myristate, magnesium myristate, zinc cetylphosphate, calcium cetylphosphate, zinc sodium cetylphosphate and so on.

Examples of a colored pigment usable herein include inorganic red pigments, such as iron oxide, iron hydroxide and iron titanate; inorganic brown pigments, such as γ-iron oxide; inorganic yellow pigments, such as iron oxide yellow and loess; inorganic black pigments, such as iron oxide black and carbon black; inorganic violet pigments, such as manganese violet and cobalt violet; inorganic green pigments, such as chromium hydroxide, chromium oxide, cobalt oxide and cobalt titanate; inorganic blue pigments, such as Prussian blue and ultramarine blue; lakes of tar pigments; lakes of natural dyes; synthetic resin powder complexes of the inorganic pigments as recited above; and so on.

Examples of a pearl pigment usable herein include titanium oxide-coated mica, titanium oxide-coated mica, bismuth oxychloride, titanium oxide-coated bismuth oxychloride, titanium oxide-coated talc, fish scales, and titanium oxide-coated colored mica; and examples of a metallic powder pigment include aluminum powder, copper powder, stainless powder, and the like.

Usable tar pigments include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, Orange No. 207 and so on; and usable natural pigments include powders of carminic acid, laccaic acid, carthamin, bradilin, crocin, and so on.

Additionally, powders formed into complexes or treated with general oil, silicone oil, a fluorine-containing compound or a surfactant to the extent not to adversely affect the present effect may be used, too. The powders as recited above can be used as a mixture of two or more thereof, if desired. The suitable proportion of the powders as recited above in total cosmetic material is from 0.1 to 99 % by weight. In the special cases where the cosmetic materials are in powder or cake form, it is appropriate that the powders account for 80 to 99 weight % of the total cosmetic material.

In the present cosmetic materials, one or more of a surfactant can also be mixed as Component (G) if needed. The surfactant mixed therein has no particular restriction, but it may be any of anionic, cationic, nonionic and ampho-ionic ones so long as they have hitherto been used in general cosmetics.

Examples of a anionic surfactant usable herein include fatty acid soap, such as sodium stearate or triethanolamine palmitate; alkyl ether carboxylic acids and salts thereof; salts of amino acid-fatty acid condensates; alkanesulfonates; alkenesulfonates; sulfonated fatty acid esters; sulfonated fatty acid amides; sulfonates of formaldehyde condensate type; alkylsulfates; higher secondary alcohol sulfates; alkyl and aryl ether sulfates; fatty acid ester sulfates, fatty acid alkylolamide sulfates; sulfates, such as Turkeky red oil; alkyl phosphates; ether phosphates; alkyl aryl ether phosphates; amide phosphates; active agents of N-acylamino acid type; and so on. Examples of a cationic surfactant usable herein include amine salts, such as alkylamine salts, polyamines and aminoalcohol fatty acid derivatives; quaternary alkylammonium salts, quaternary arylammonium salts, pyridinium salts and imidazolium salts; and so on.

Examples of a nonionic surfactant usable herein include sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkyl ethers, polyoxypropylene alkyl ethers, polyoxyethylene alkyl phenyl ethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ehter, polyoxyethylene phytosterol ether, polyoxyethylene cholestanol ether, polyoxyethylene cholesteryl ether, straight-chain or branched polyoxyalkylene-modified organopolysiloxanes, organopolysiloxanes modified with both straight-chain or branched polyoxyalkylene and alkyl groups, alkanolamides, sugar ethers, sugar amides and so on; and examples of an ampho-ionic surfactant usable herein include surfactants of betaine, aminocarboxylate, imdazoline derivative, amidoamine and like types. The suitable proportion of surfactants in total cosmetic material is from 0.1 to 20 %, particularly from 0.2 to 10 %, by weight.

The present cosmetic materials can further contain cross-linked organopolysiloxanes as Component (H), if desired. The cross-linked organopolysiloxanes suitable for the present cosmetic materials are those which cause swelling when they contain a silicone having low viscosity of from 0.65 to 10.0 mm²/sec (25°) in a quantity larger than their weight. Further, it is desirable that the cross-linked structure of those organopolysiloxanes be formed by reaction between the hydrogen atoms bonded directly to silicon atoms and a cross-linking agent having at least two vinylic reactive moieties per molecule. Also, it is possible to use cross-linked organopolysiloxanes containing in each individual cross-linked molecules at least one kind of moiety selected from the group consisting of polyoxyalkylene, alkyl, alkenyl, aryl and fluoroalkyl moieties. The suitable amount of such cross-linked organopolysiloxanes mixed in the present cosmetic material is from 0.1 to 50 %, preferably from 1 to 30 %, of the total weight of the cosmetic material.

The present cosmetic materials can further contain one or more of silicone resin as Component (I), if desired.

Silicone resins appropriately used herein are acrylsilicone resins, such as acryl-silicone graft or block copolymers. Further, it is possible to use an acrylsilicone resin containing in a molecule at least one moiety selected from the group consisting of a pyrrolidone moiey, a long-chain alkyl moiety, a polyoxyalkylene moiety, a fluoroalkyl moiety and anionic moieties, such as carboxylic acid.

Other silicone resins favorably used herein are reticular silicone compounds represented by MQ, MDQ, MT, MDT or MDTQ. The characters M, D, T and Q used herein stand for well-known basic structural units referred to as M units, D units, T units and Q units, respectively. In addition, reticular silicone compounds having in a molecule at least one moiety selected from the group consisting of pyrrolidone, long-chain alkyl, polyoxyalkylene, fluoroalkyl and amino moieties can also be used herein.

When the silicone resins, such as acrylsilicone resins and reticular silicone compounds, are mixed in the present cosmetic materials, the appropriate proportion of silicone resins mixed is from 0.1 to 20 weight %, particularly from 1 to 10 weight %, to the total cosmetic material.

To the present cosmetic materials, the ingredients used in general cosmetic materials, such as oil-soluble gelling agents, clay minerals modified with organic compounds, resins, antiperspirants, ultraviolet absorbents, ultraviolet absorbing-scattering agents, moisture retention agents, antiseptics, antimicrobial agents, perfume, salts, antioxidants, pH regulators, chelating agents, refrigerants, anti-inflammatory agents, skin beautifying ingredients (a skin whitener, a cell activator, a rough dry skin improver, a blood circulation promoter, a skin astringent and an anti-seborrheic agent), vitamins, amino acids, nucleic acids, hormones, clathrate compounds and hair setting agents, can be added so far as they have no adverse influence on the effects of the present invention.

Examples of an oil-soluble gelling agent which can be added include metal soaps, such as aluminum stearate, magnesium stearate and zinc myristate; amino acid derivatives, such as N-lauroyl-L-glutamic acid and α,γ-di-n-butylamine; dextrin fatty acid esters, such as dextrin palmitic acid ester, dextrin stearic acid ester and dextrin 2-ethylhexaminic acid palmitic acid ester; sucrose fatty acid esters, such as sucrose palmitic acid ester and sucrose stearic acid ester; benzylidene derivatives of sorbitol, such as monobenzylidene sorbitol and dibenzylidene sorbitol; clay minerals modified with organic compounds, such as dimethylbenzyldodecyl ammonium montmorillonite clay and dimethyldioctadecyl ammonium montmorillonite clay; and so on.

Examples of an antiperspirant which can be added include aluminum chlorohydrate, aluminum chloride, aluminum sesquichlorohydrate, zirconium hydroxychloride, aluminum zirconium hydroxychloride, aluminum-zirconium-glycine complex and so on.

Examples of an ultraviolet absorbent which can be added include ultraviolet absorbents of benzoic acid type, such as include ultraviolet absorbents of benzoic acid type, such as p-aminobenzoic acid; those of anthranilic acid type, such as methyl anthranilate; those of salicylic acid type, such as methyl salicylate; those of succinic acid type, such as octyl p-methoxysuccinate; those of benzophenone type, such as 2,4-dihydroxybenzophenone; those of urocanic acid type, such as ethyl urocanate; those of dibenzoylmethane type, such as 4-t-butyl-4'-methoxydibenzoylmethane; and so on: and examples of an ultraviolet absorbing-scattering agent which can be added include powders capable of absorbing and scattering ultraviolet rays, such as particulate titanium dioxide, particulate Fe-doped titanium dioxide, particulate zinc oxide, particulate cerium oxide, composites of these oxides, and so on.

Examples of a moisture retention agent which can be added include glycerin, sorbitol, propylene glycol, dipropylene glycol, 1,3-butylene glycol, glucose, xylitol, maltitol, polyethylene glycol, hyaluronic acid, chondroitin sulfuric acid, pyrrolidone carboxylic acid, polyoxyethylene methylglucoside, polyoxypropylene methylglucoside, and so on.

Examples of an antimold-antiseptic agent which can be added include alkyl p-hydroxybenzoates, benzoic acid, sodium benzoate, sorbic acid, potassium sorbate, phenoxyethanol, and so on; and examples of an antibacterial agent which can be added include benzoic acid, salicylic acid, carbolic acid, sorbic acid, alkyl p-hydroxybenzoates, p-chlorometacresol, hexachlorophene, benzalkonium chloride, chlorhexidine chloride, trichlorocarbanilide, photosensitizers, phenoxyethanol, and so on.

Examples of an antioxidant which can be added include tocopherol, butylhydroxyanisole, dibutylhydroxytoluene, phytic acid and so on; examples of a pH regulator which can be added include lactic acid, citric acid, glycolic acid, succinic acid, tartaric acid, dl-malic acid, potassium carbonate, sodium hydrogen carbonate, ammonium hydrogen carbonate and so on; examples of a chelating agent which can be added include alanine, sodium ethylenediaminetetraacetate, sodium polyphosphate, sodium metaphosphate, phosphoric acid and so on; examples of a refrigerant which can be added include L-menthol, camphor and so on; and examples of an anti-inflammatory agent which can added include allantoin, glycyrrhizin and salts thereof, glycyrrhetinic acid and stearyl glycyrrhetinate, tranexamic acid, azulene, and so on.

Examples of skin-beautifying ingredients which can be added include whitening agents, such as placenta extract, arbutin, glutathione and Yukinoshita extract; cell activators, such as royal jelly, photosensitizers, cholesterol derivatives and calf blood extract; rough dry skin improvers; blood circulation improvers, such as nonylic acid vanillyl amide, benzyl nicotinate, β-butoxyethyl nicotinate capsaicin, zingerone, cantharis tincture, ichtammol, caffeine, tannic acid, α-borneol, tocopheryl nicotinate, inositol hexanicotinate, cyclandelate, cinnarizine, tolazoline, acetyl choline, verapamil, cepharanthin and γ-oryzanol; skin astringents, such as zinc oxide and tannic acid; anti-seborrheic agents, such as sulfur and thianthol; and so on.

Examples of vitamins which can be added include vitamin A, such as vitamin A oil, retinol, retinyl acetate and retinyl palmitate; vitamin B, including vitamin B₂ such as riboflavin, riboflavin butyrate and flavin adenine nucleotide, vitamin B₆ such as pyridoxine hydrochloride, pyridoxine dioctanoate and pyridoxine tripalmitate, vitamin B₁₂ and its derivatives, vitamin B₁₅ and its derivatives, and so on; vitamin C, such as L-ascorbic acid, L-ascorbic acid dipalmitic ester, sodium (L-ascorbic acid)-2-sulfate and dipotassium L-ascorbic acid diphosphate; vitamin D, such as ergocalciferol and cholecarciferol; vitamin E, such as α-tocopherol, β-tocopherol, γ-tocopherol, dl-a-tocopheryl acetate, dl-α-tocopheryl nicotinate and dl-α-tocopheryl succinate; vitamin H; vitamin P; nicotinic acids, such as nicotinic acid, benzyl nicotinate and nicotinic acid amide; pantothenic acids, such as calcium pantothenate, D-pantothenyl alcohol, pantothenyl ethyl ether and acetylpantothenyl ethyl ether; biotin; and the like.

Examples of an amino acid which can be added include glycine, valine, leucine, isoleucine, serine, threonine, phenylaranine, alginine, lysine, aspartic acid, glutamic acid, cystine, cysteine, methionine, tryptophan and the like; examples of a nucleic acid which can be added include deoxyribonucleic acid and the like; and examples of hormone which can be added include estradiol, ethenyl estradiol, and the like.

As examples of a hair setting agent which can be added, mention may be made of ampho-ionic, anionic, cationic and nonionic polymers. More specifically, such polymers include macromolecular compounds of polyvinyl pyrrolidone type, such as polyvinyl pyrrolidone and vinyl pyrrolydone-vinyl acetate copolymer; macromolecular compounds of acidic vinyl ether type, such as copolymer of methyl vinyl ether and maleic anhydride alkyl halfester; high polymers of acidic polyvinyl acetate type, such as vinyl acetate/crotonic acid copolymer; acidic acrylic macromolecular compounds, such as (meth)acrylic acid/alkyl (meth)acrylate copolymers and (meth)acrylic acid/alkyl (meth)acrylate/alkylacrylamide copolymers; and ampho-ionic acrylic macromolecular compounds, such as N-methacryloylethyl-N, N-dimethylammonium·α-N-methylcarboxy-betaine/alkyl (meth)acrylate copolymers and hydroxypropyl (meth)acrylate/butylaminoethyl methacrylate/acrylic acid octylamide copolymer. In addition, polymers of natural origin, such as cellulose or derivatives thereof, keratin and collagen or derivatives thereof, can also be used appropriately.

The term "cosmetic material" as used in the invention is intended to include skin-care cosmetics, such as face lotion, milky lotion, cream, cleansing cream, face pack, oil liquid, massage articles, toilet soap, detergent, hand cream and lip balm; makeup cosmetics, such as makeup foundation, face powder, liquid foundation, oil foundation, rouge, eye shadow, mascara, eyeliner, eyebrow and lipstick; hairdressing articles, such as shampoo, rinse, treatment and a set agent; antiperspirants; UV protective cosmetics, such as sunscreen lotion and sun block cream; and so on.

Additionally, the present cosmetic material may have any of forms, including liquid, emulsion, cream, solid, paste, gel, powder, pressed cake, multilayer, moose, spray, stick and pencil forms, if desired. In particular, solid and stick forms are suitable for the present cosmetic material.

### Examples

The present invention will now be illustrated in greater detail by reference to the following examples. However, the invention should not be construed as being limited to these examples. Additionally, the term "%" used hereinafter means "% by weight" unless noted otherwise.

### Synthesis Example 1

In a flask were placed 280 g of maleic anhydride-modified low-molecular-weight polyethylene wax (Mitsui Hiwax 3202A, trade name, a product of Mitsui Chemical) having a molecular weight of 4,400, an acid value of 20, a carboxyl equivalent of 2,800 and a melting point of 102°C (wherein 0.1 mole of carboxyl groups were contained), 37.2 g (0.3 mole) of vinylcyclohexene epoxide and 640 g of xylene. Reaction was run for 5 hours in the flask while refluxing the xylene. After the conclusion of the reaction, both xylene and excess vinylcyclohexene epoxide were distilled off by heating under reduced pressure, and polyethylene wax having a vinyl group in individual molecules thereof was obtained. Then, 200 g of the thus obtained polyethylene wax was weighed out, mixed with 200 g of one-end hydrogensiloxane having the following average structural formula, 800 g of xylene and 0.2 g of a 3 % isopropanol solution of chloroplatinic acid in a flask, and therein the reactants underwent 5-hour reaction under reflux of xylene.

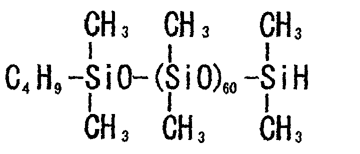

By heating and distilling off the solvent under reduced pressure, silicone-modified Wax (I) having a melting point of 98°C was obtained.

### Synthesis Example 2

In a flask were placed 280 g of maleic anhydride-modified low-molecular-weight polypropylene wax (Mitsui Hiwax NP0555A, trade name, a product of Mitsui Chemical) having a molecular weight of 17,400, an acid value of 47, a carboxyl equivalent of 1,190 and a melting point of 140°C (wherein 0.1 mole of carboxyl groups were contained), 119 g (0.047 mole in terms of epoxy group) of silicone having an epoxy group at one molecular-chain end and an average structural formula illustrated below (epoxy equivalent: 2,500), 0.5 g of dimethylethanolamine as a catalyst and 400 g of xylene as a solvent.

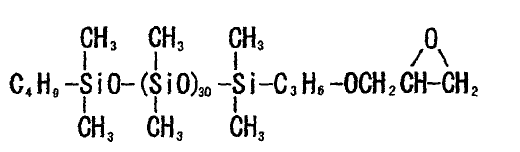

And in the flask, reaction was run for 5 hours while refluxing the xylene. By heating and distilling off the solvent under reduced pressure, silicone-modified Wax (II) having a melting point of 127°C was obtained.

| Example 1: Milky Lotion | |
|---|---|
| Ingredients | Amount mixed (weight %) |
| 1. Silicone-modified wax (I) obtained in Synthesis Example 1 | 2.0 |
| 2. Dimethylpolysiloxane (6 mm²/sec at 25°C) | 30.0 |
| 3. Decamethylcyclopentasiloxane | 10.0 |
| 4. Glyceryl trioctanoate | 5.0 |
| 5. Polyether-modified silicone (note 1) | 5.0 |
| 6. 1,3-Butylene glycol | 5.0 |
| 7. Antiseptic | proper |
| 8. Perfume | proper |
| 9. Purified water | 43.0 |
| (note 1) : KF-6017 (trade name, a product of Shin-Etsu Chemical Co., Ltd. | |

### [Preparation Process]

(A) The ingredients 1 to 5 were mixed together under heating to prepare a solution.
(B) The ingredients 6, 7 and 9 were mixed together, and then admixed with the solution obtained in the step (A) to prepare an emulsion.
(C) The emulsion obtained in the step (B) was cooled, and admixed with the ingredient 8 to prepare milky lotion.

The milky lotion thus prepared in accordance with the invention had no tackiness, and spread easily and smoothly. In addition, it was confirmed that the users of this lotion had not only a feeling that the lotion was clung to the skin and well held by the skin but also a lustrous finish on the skin.

| Example 2: Cream of Water-in-Oil Type | |
|---|---|
| Ingredients | Amount mixed (weight %) |
| 1. Dimethylpolysiloxane (6 mm²/sec at 25°C) | 10.0 |
| 2. Decamethylcyclopentasiloxane | 7.0 |
| 3. Glyceryl trioctanoate | 5.0 |
| 4. Polyether-modified branched-type silicone (note 1) | 2.0 |
| 5. Silicone-modified wax (I) obtained in Synthesis Example 1 | 1.0 |
| 6. Dipropylene glycol | 7.0 |
| 7. Antiseptic | proper |
| 8. Perfume | proper |
| 9. Purified water | 68.0 |
| (note 1) : KF-6028 (trade name, a product of Shin-Etsu Chemical Co., Ltd.) | |

### [Preparation Process]

(A) The ingredients 1 to 5 were mixed together under heating to prepare a solution.
(B) The ingredients 6 to 9 were mixed together, and then added to the solution obtained in the step (A), and further emulsified with stirring.

The water-in-oil cream thus prepared in accordance with the invention had neither-oiliness nor tackiness, spread easily and smoothly, and gave a light feel to users' skin. In addition, it was confirmed that the users of this cream had not only a feeling that the cream was clung to the skin and well held by the skin but also a lustrous finish on the skin.

| Example 3: Cream of Water-in-Oil Type | |
|---|---|
| Ingredients | Amount mixed (weight %) |
| 1. Silicone-modified wax (I) obtained in Synthesis Example 1 | 2.0 |
| 2. Dimethylpolysiloxane (6 mm²/sec at 25°C) | 10.0 |
| 3. Cross-linked polyether-modified silicone (note 1) | 5.0 |
| 4. Dipropylene glycol | 10.0 |
| 5. Sodium citrate | 0.2 |
| 6. Ethanol | 5.0 |
| 7. Antiseptic | proper |
| 8. Perfume | proper |
| 9. Purified water | 67.8 |
| (note 1) : KSG-21 (trade name, a product of Shin-Etsu Chemical Co., Ltd.) | |

### [Preparation Process]

(A) The ingredients 1 to 3 were mixed together under heating to prepare a solution.
(B) The ingredients 4 to 9 were mixed together, and then added to the solution obtained in the step (A), and further emulsified with stirring.

The water-in-oil cream thus prepared in accordance with the invention had neither oiliness nor tackiness, spread easily and smoothly, and gave a light and fresh feel to users' skin. In addition, it was confirmed that the users of this cream had not only a feeling that the cream way clung to the skin and well held by the skin but also a matte finish on the skin.

| Example 4: Makeup Foundation of Water-in-Oil Type | |
|---|---|
| Ingredients | Amount mixed (weight %) |
| 1. Cross-linked polyether-modified silicone (note 1) | 4.0 |
| 2. Cross-linked dimethylpolysiloxane (note 2) | 1.0 |
| 3. Polyether-modified silicone (note 3) | 0.5 |
| 4. Dimethylpolysiloxane (6 mm²/sec at 25°C) | 6.0 |
| 5. Dimethylpolysiloxane (20 mm²/sec at 25°C) | 2.0 |
| 6. Decamethylcyclopentasiloxane | 3.0 |
| 7. Titanium oxid-cyclopentasiloxane dispersion (note 4) | 10.0 |
| 8. Silicone-modified wax (I) obtained in Synthesis Example 1 | 1.0 |
| 9. Dipropylene glycol | 5.0 |
| 10. Sodium citrate | 0.2 |
| 11. Methyl cellulose (2% aq. soln.) (note 5) | 2.5 |
| 12. Ethanol | 3.0 |
| 13. Antiseptic | proper |
| 14. Perfume | proper |
| 15. Purified water | 61.8 |
| (note 1) : KSG-21 (trade name, a product of Shin-Etsu Chemical Co., Ltd.) | |
| (note 2) : KSG-15 (trade name, a product of Shin-Etsu Chemical Co., Ltd.) | |
| (note 3) : KF-6017 (trade name, a product of Shin-Etsu Chemical Co., Ltd.) | |
| (note 4) : SPD-T1S (trade name, a product of Shin-Etsu Chemical | |
| Co., Ltd.) | |
| (note 5): Metholose 65-SH4000 (trade name, a product of Shin-Etsu-Chemical Co., Ltd.) | |

### [Preparation Process]

(A) The ingredients 1 to 8 were mixed together under heating.
(B) The ingredients 9 to 15 were mixed together and dissolved, and then added to the mixture obtained in the step (A), and further emulsified with stirring.

The water-in-oil makeup foundation thus prepared in accordance with the invention had neither oiliness nor tackiness, spread easily and smoothly, and gave a light and fresh feel to users' skin. In addition, it was confirmed that the users of this foundation had not only a feeling that the cream was clung to the skin and well held by the skin but also a matte finish on the skin, and further the foundation had UV cut effect and permitted makeup effect to last long.

| Example 5: Cream of Oil-in-Water Type | |
|---|---|
| Ingredients | Amount mixed (weight %) |
| 1. Silicone-modified wax (I) obtained in Synthesis Example 1 | 2.0 |
| 2. Cross-linked dimethylpolysiloxane (note 1) | 15.0 |
| 3. Decamethylcyclopentasiloxane | 10.0 |
| 4. Dimethylpolysiloxane (6 mm²/sec at 25°C) | 18.0 |
| 5. Polyether-modified silicone (note 2) | 0.7 |
| 6. Propylene glycol | 3.0 |
| 7. Polyacrylamide mixture (note 3) | 0.8 |
| 8. Xanthan gum (2% aq. soln.) | 8.0 |
| 9. Antiseptic | proper |
| 10. Perfume | proper |
| 11. Purified water | 42.5 |
| (note 1) : KSG-16 (trade name, a product of Shin-Etsu Chemical Co., Ltd.) | |
| (note 2) : KF-6011 (trade name, a product of Shin-Etsu Chemical Co., Ltd.) | |
| (note 3): SEPIGEL 305 (trade name, a product of SEPIC) | |

### [Preparation Process]

(A) The ingredients 1 to 4 were mixed together under heating.
(B) The ingredients 5 to 11 were mixed and dissolved.
(C) The mixture obtained in the step (A) was added to the solution obtained in the step (B), and then emulsified with stirring.

It was confirmed that the oil-in-water cream thus prepared in accordance with the invention had excellent properties of spreading smoothly and giving a light feel to users' skin.

| Example 6: Solid Cream of Water-in-Oil Type | |
|---|---|
| Ingredients | Amount mixed (weight %) |
| 1. Silicone-modified wax (I) obtained in Synthesis Example 1 | 30.0 |
| 2. Dimethylpolysiloxane (6 mm²/sec at 25°C) | 24.0 |
| 3. Decamethylcyclopentasiloxane | 24.0 |
| 4. Polyether-modified silicone (note 1) | 2.0 |
| 5. 1,3-Butylene glycol | 2.0 |
| 6. Antiseptic | proper |
| 7. Perfume | proper |
| 8. Purified water | 18.0 |
| (note 1) : KF-6017 (trade name, a product of Shin-Etsu Chemical Co., Ltd.) | |

### [Preparation Process]

(A) The ingredients 1 to 4 were mixed together under heating to prepare a solution.
(B) The ingredients 5 to 8 were mixed and dissolved, and then admixed with the solution obtained in the step (A) to prepare an emulsion.
(C) The emulsion obtained in the step (B) was charged into products.

The solid cream thus prepared in accordance with the invention had excellent properties of being free of both oiliness and tackiness although it contained high proportions of unctuous agents, spreading easily and smoothly, and giving a light feeling to users' skin. In addition, it was confirmed that the users of this solid cream had a feeling that the cream was clung to the skin and well held by the skin.

| Example 7: Lipstick | |
|---|---|
| Ingredients | Amount mixed (weight %) |
| 1. Silicone-modified wax (I) obtained in Synthesis Example 1 | 40.0 |
| 2. Dimethylpolysiloxane (6 mm²/sec at 25°C) | 30.0 |
| 3. Decamethylcyclopentasiloxane | 26.0 |
| 4. Acrylsilicone resin (note 1) | 4.0 |
| 5. Pigments | proper |
| 6. Antiseptic | proper |
| 7. Perfume | proper |
| (note 1): KP-545 (trade name, a product or Shin-Etsu Chemical Co., Ltd. | |

### [Preparation Process]

(A) The ingredients 1 to 3 were mixed together under heating to prepare a solution.
(B) The ingredients 4 to 6 were mixed together to prepare a dispersion, and then admixed homogeneously with the solution obtained in the step (A).
(C) The ingredient 7 was added to the admixture obtained in the step (B), and packed into products.

The lipstick thus prepared in accordance with the invention had neither oiliness nor tackiness, and spread easily and smoothly. In addition, it was confirmed that the lipstick applied to lips stuck well and lasted long.

| Example 8: Powder Foundation | |
|---|---|
| Ingredients | Amount mixed (weight %) |
| 1. Sericite | 40.0 |
| 2. Mica | 10.0 |
| 3. Talc | the rest |
| 4. Titanium dioxide | 10.0 |
| 5. Particulate titanium dioxide | 5.0 |
| 6. Magnesium stearate | 3.0 |
| 7. Pigment | 4.2 |
| 8. Silicone-modified wax (I) obtained in Synthesis Example 1 | 1.0 |
| 9. Dimethylpolysiloxane (6 mm²/sec at 25°C) | 3.0 |
| 10. Antiseptic | proper |
| 11. Perfume | proper |

### [Preparation Process]

(A) The ingredients 8 to 11 were mixed together.
(B) The ingredients 1 to 7 were mixed together, and then admixed homogeneously with the mixture obtained in the step (A).
(C) The homogeneous mixture obtained in the step (B) was charged into metal pans and underwent press forming.

The powder foundation thus formed in accordance with the invention had no tackiness and spread lightly. In addition, it was confirmed that the powder foundation obtained stayed well due to its good adhesion and gave a lustrous finish on the skin.

| Example 9: Cream Foundation | |
|---|---|
| Ingredients | Amount mixed (weight %) |
| 1. Cross-linked polyether-modified silicone (note 1) | 4.0 |
| 2. Gryceryl trioctanoate | 3.0 |
| 3. Dimethylpolysiloxane (6 mm²/sec at 25°C) | 5.0 |
| 4. Decamethylcyclopentasiloxane | 6.0 |
| 5. Silicone-modified wax (I) obtained in Synthesis Example 1 | 2.0 |
| 6. Fluorine-modified hybrid silicone complex powder (note 2) | 2.5 |
| 7. Pigment | 8.0 |
| 8. Acrylsilicone resin (note 3) | 5.0 |
| 9. Dipropylene glycol | 5.0 |
| 10. Sodium citrate | 0.2 |
| 11. Antiseptic | proper |
| 12. Perfume | proper |
| 13. Purified water | 59.3 |
| (note 1) : KSG-21 (trade name, a product of Shin-Etsu Chemical Co., Ltd.) | |
| (note 2) : KSP-200 (trade name, a product of Shin-Etsu Chemical Co., Ltd.) | |
| (note 3): KP-545 (trade name, a product of Shin-Etsu Chemical Co., Ltd.) | |

### [Preparation Process]

(A) The ingredients 1 to 6 were mixed together under heating.
(B) The ingredients 9 to 13 were mixed and dissolved, and then added to the solution obtained in the step (A), and further emulsified with stirring.
(C) The ingredients 7 and 8 were mixed together, and added to the emulsion obtained in the step (B) and homogenized.

The cream foundation thus prepared in accordance with the invention had no tackiness, and spread easily and smoothly. In addition, it was confirmed that this cream foundation stayed well on the skin due to its good adhesion and ensured a matte finish feel.

| Example 10: Solid Foundation | |
|---|---|
| Ingredients | Amount mixed (weight %) |
| 1. Silicone-modified wax (I) obtained in Synthesis Example 1 | 30.0 |
| 2. Polyethylene wax | 5.0 |
| 3. Dimethylpolysiloxane (6 mm²/sec at 25°C) | 32.5 |
| 4. Decamethylcyclopentasiloxane | 28.5 |
| 5. Acrylsilicone resin (note 1) | 4.0 |
| 6. Pigments | proper |
| 7. Antiseptic | proper |
| 8. Perfume | proper |
| (note 1) : KP-545 (trade name, a product of Shin-Etsu Chemical Co., Ltd. | |

### [Preparation Process]

(A) The ingredients 1 to 4 were mixed together under heating to prepare a solution.
(B) The ingredients 5 to 7 were mixed together to prepare a dispersion, and then admixed homogeneously with the solution obtained in the step (A).
(C) The ingredient 8 was added to the admixture obtained in the step (B), and packed into products.

The solid foundation thus prepared in accordance with the invention had neither oiliness nor tackiness, and spread easily and smoothly. In addition, it was confirmed that the foundation applied to the skin stuck well and lasted long.

| Example 11: Compact Foundation of Water-in-Oil Type | |
|---|---|
| Ingredients | Amount mixed (weight %) |
| 1. Silicone-modified wax (I) obtained in Synthesis Example 1 | 30.0 |
| 2. Dimethylpolysiloxane (6 mm²/sec at 25°C) | 24.0 |
| 3. Decamethylcyclopentasiloxane | 22.0 |
| 4. Acrylsilicone resin (note 1) | 4.0 |
| 5. Trimethylsiloxysilicate (note 2) | 1.0 |
| 6. Polyether-modified silicone (note 3) | 2.0 |
| 7. Pigment | proper |
| 8. 1,3-Butylene glycol | 2.0 |
| 9. Antiseptic | proper |
| 10. Perfume | proper |
| 11. Purified water | 15.0 |
| (note 1) : KP-545 (trade name, a product of Shin-Etsu Chemical Co., Ltd.) | |
| (note 2): KF-7312J (trade name, a product of Shin-Etsu Chemical Co., Ltd.) | |
| (note 3) : KF-6017 (trade name, a product of Shin-Etsu Chemical Co., Ltd.) | |

### [Preparation Process]

(A) The ingredients 1 to 5 were mixed together under heating to prepare a solution.
(B) The ingredients 8, 9 and 11 were mixed and dissolved, and then admixed with the solution obtained in the step (A), and further made into an emulsion with stirring.
(C) The ingredient 7 was mixed with the ingredient 6 to prepare a dispersion, and then admixed homogeneously with the emulsion obtained in the step (B) with stirring.
(D) The ingredient 10 was added to the emulsion obtained in the step (C), and then charged into products.

The water-in-oil compact foundation thus prepared in accordance with the invention had excellent properties of being free of both oiliness and tackiness although it contained high proportions of unctuous agents, spreading easily and smoothly, and giving a light feeling to users' skin. In addition, it was confirmed that this compact foundation gave users a feeling that the foundation was clung to the skin and well held by the skin and ensured long-lasting makeup effect.

| Example 12: Eye Shadow | |
|---|---|
| Ingredients | Amount mixed (weight %) |
| 1. Sericite | 40.0 |
| 2. Mica | 10.0 |
| 3. Talc | the rest |
| 4. Titanium dioxide | 10.0 |
| 5. Particulate titanium dioxide | 5.0 |
| 6. Magnesium stearate | 3.0 |
| 7. Pigment | proper |
| 8. Octyl dodecanol | 3.0 |
| 9. Dimethylpolysiloxane (6 mm²/sec at 25°C) | 8.0 |
| 10. Silicone-modified wax (I) obtained in Synthesis Example 1 | 2.0 |
| 11. Antiseptic | proper |
| 12. Perfume | proper |

### [Preparation Process]

(A) The ingredients 8 to 11 were mixed together under heating.
(B) The ingredients 1 to 7 were mixed together, and then admixed homogeneously with the mixture obtained in the step (A).
(C) The ingredient 14 was added to the mixture obtained in the step (B).

The eye shadow thus prepared in accordance with the invention was free of tackiness and spread lightly. In addition, it was confirmed that this eye shadow stayed well due to its good adhesion, gave a lustrous finish on the skin, and ensured long-lasting makeup effect.

| Example 13: Powdered Eyebrow | |
|---|---|
| Ingredients | Amount mixed (weight %) |
| 1. Silicone-modified wax (I) obtained in Synthesis Example 1 | 3.0 |
| 2. Dimethylpolysiloxane (6 mm²/sec at 25°C) | 5.0 |
| 3. Glyceryl trioctanoate | 2.0 |
| 4. Silicone-treated mica | 40.0 |
| 5. Silicone-treated barium sulfate | 15.0 |
| 6. Silicone-treated titanium dioxide | 10.0 |
| 7. Silicone-treated pigment | proper |
| 8. Hybrid silicone complex powder (note 1) | 1.5 |
| 9. Spherical polymethylsilsesquioxane powder (note 2) | 2.5 |
| 10. Silicone-treated talc | the rest |
| 11. Antiseptic | proper |
| 12. Perfume | proper |
| (note 1) : KSP-100 (trade name, a product of Shin-Etsu Chemical Co., Ltd.) | |
| (note 2) : KMP-590 (trade name, a product of Shin-Etsu Chemical Co., Ltd.) | |

### [Preparation process]

(A) The ingredients 4 to 12 were mixed homogeneously.
(B) The ingredients 1 to 3 were mixed and dissolved, and then admixed homogeneously with the mixture obtained in the step (A).
(C) The homogeneous mixture obtained in the step (B) was subjected to press forming in metal pans.

The powdered eyebrow thus formed in accordance with the invention was free of tackiness and spread lightly. In addition, it was confirmed that this powdered eyebrow stayed well due to its good adhesion, gave a lustrous finish on brows, and ensured long-lasting makeup effect.

| Example 14: Pencil-shaped Eyebrow | |
|---|---|
| Ingredients | Amount mixed (weight %) |
| 1. Silicone-modified wax (I) obtained in Synthesis Example 1 | 40.0 |
| 2. Polyethylene wax | 10.0 |
| 3. Dimethylpolysiloxane (6 mm²/sec at 25°C) | 40.0 |
| 4. Decamethylcyclopentasiloxane | 5.0 |
| 5. Acrylsilicone resin (note 1) | 5.0 |
| 6. Pigments | proper |
| 7. Antiseptic | proper |
| 8. Perfume | proper |
| (note 1): KP-545 (trade name, a product of Shin-Etsu Chemical Co., Ltd. | |

### [Preparation Process]

(A) The ingredients 1 to 4 were mixed together under heating to prepare a solution.
(B) The ingredients 5 to 7 were mixed together to prepare a dispersion, and then added to the solution obtained in the step (A) , and further stirred homogeneously.
(C) The ingredient 8 was added to the admixture obtained in the step (B), and packed in a pencil shape.

It was confirmed that the pencil-shaped eyebrow thus prepared in accordance with the invention caused no powdery blur, slid well, and had long-lasting makeup effect.

| Example 15: Hair Cream | |
|---|---|
| Ingredients | Amount mixed (weight %) |
| 1. Silicone-modified wax (I) obtained in Synthesis Example 1 | 2.0 |
| 2. Dimethylpolysiloxane (6 mm²/sec at 25°C) | 5.0 |
| 3. Decamethylcyclopentasiloxane | 8.0 |
| 4. Chlorostearyltrimethylammonium | 1.5 |
| 5. Glycerol | 3.0 |
| 6. Propylene glycol | 5.0 |
| 7. Hydroxyethyl cellulose | 0.2 |
| 8. Antiseptic | proper |
| 9. Perfume | proper |
| 10. Purified water | 75.3 |

### [Preparation Process]

(A) The ingredients 1 to 3 were mixed together under heating to prepare a solution.
(B) The ingredients 4 to 8 and 10 were mixed homogeneously into a solution.
(C) The solution obtained in the step (B) was added to the solution obtained in the step (A), and emulsified. The emulsion obtained was cooled, and admixed with the ingredient 9.

The hair cream thus prepared in accordance with the invention spread well when applied to hair and gave hair satisfactory softness, smoothness, setting effect, moist feel and fine luster. In other words, the composition prepared herein proved to be totally excellent for hair cream.

| Example 16: Conditioning Mousse | |
|---|---|
| Ingredients | Amount mixed (weight %) |
| 1. Silicone-modified wax (I) obtained in Synthesis Example 1 | 0.5 |
| 2. Dimethylpolysiloxane (6 mm²/sec at 25°C) | 2.0 |
| 3. Cross-linked dimethylpolysiloxane (note 1) | 0.5 |
| 4. Glyceryl trioctanoate | 1.5 |
| 5. Glycerol | 3.0 |
| 6. Chlorostearyltribenzylammonium | 0.5 |
| 7. Polyoxyethylene hydrogenated castor oil | 0.5 |
| 8. Ethanol | 7.0 |
| 9. Antiseptic | proper |
| 10. Perfume | proper |
| 11. Purified water | the rest |
| 12. Liquefied petroleum gas | 5.0 |
| (note 1) : KSG-16 (trade name, a product of Shin-Etsu Chemical Co., Ltd. | |

### [Preparation Process]

(A) The ingredients 1 to 4 were mixed together under heating to prepare a solution.
(B) The ingredients 5 to 9 and 11 were mixed into a solution.
(C) The solution obtained in the step (B) was added to the solution obtained in the step (A), and emulsified. The emulsion obtained was cooled, and admixed with the ingredient 10.
(D) The emulsion obtained in the step (C) was charged into aerosol cans, and thereto the ingredient 12 was added.

The conditioning mousse thus prepared in accordance with the invention had excellent feels, including satisfactory moist, soft and smooth feels, in addition to no oily feel. Further, it was confirmed that this mousse stayed well due to its good adhesion and ensured a matte finish feel.

| Example 17: Antiperspirant of Roll-on Type | |
|---|---|
| Ingredients | Amount mixed (weight %) |
| 1. Silicone-modified wax (I) obtained in Synthesis Example 1 | 5.0 |
| 2. Cross-linked polyether-modified silicone (note 1) | 20.0 |
| 3. Dimethylpolysiloxane (6 mm²/sec at 25°C) | 10.0 |
| 4. Cross-linked dimethylpolysiloxane (note 2) | 15.0 |
| 5. Decamethylcyclopentasiloxane | 30.0 |
| 6. Aluminum zirconium tetrachlorohydrate | 20.0 |
| 7. Perfume | proper |
| (note 1): KSG-21 (trade name, a product of Shin-Etsu Chemical Co., Ltd.) | |
| (note 2): KSG-15 (trade name, a product of Shin-Etsu Chemical Co., Ltd.) | |

### [Preparation Process]

(A) The ingredients 1 to 5 were mixed together under heating.
(B) The ingredients 6 and 7 were added to the mixture obtained in the step (A), and dispersed homogeneously.

The thus obtained roll-on antiperspirant spread smoothly, gave a refreshing feeling, and had neither tacky nor oily feel. Further, it caused no change with temperature variations and lapse of time. In other words, the antiperspirant according to the invention had excellent usability and stability.

| Example 18: Antiperspirant of Water-in-Oil Type | |
|---|---|
| Ingredients | Amount-mixed (weight %) |
| 1. Silicone-modified wax (I) obtained in Synthesis Example 1 | 2.0 |
| 2. Cross-linked polyether-modified silicone (note 1) | 7.0 |
| 3. Decamethylcyclopentasiloxane | 7.0 |
| 4. Glyceryl trioctanoate | 8.0 |
| 5. 1,3-Butylene glycol | 5.0 |
| 6. Sodium citrate | 0.2 |
| 7. Aluminum chlorohydrate | 20.0 |
| 8. Perfume | proper |
| 9. Purified water | 50.8 |
| (note 1): KSG-21 (trade name, a product of Shin-Etsu Chemical Co., Ltd.) | |

### [Preparation Process]

(A) The ingredients 1 to 4 were mixed together under heating.
(B) The ingredients 5, 6 and 9 were mixed, and further admixed with the ingredients 7 and 8 to prepare a solution.
(C) The solution obtained in the step (B) was added to the mixture obtained in the step (A), and emulsified with stirring.

The thus prepared antiperspirant of water-in-oil type spread smoothly, gave a refreshing feeling, and had neither tacky nor oily feel. In addition, it caused no change with temperature variations and lapse of time. In other words, the water-in-oil antiperspirant according to the invention had excellent usability and stability.

| Example 19: Antiperspirant of Solid Type | |
|---|---|
| Ingredients | Amount mixed (weight %) |
| 1. Silicone-modified wax (I) obtained in Synthesis Example 1 | 22.0 |
| 2. Polyethylene wax | 4.0 |
| 3. Dimethylpolysiloxane (6 mm²/sec at 25°C) | 22.0 |
| 4. Decamethylcyclopentasiloxane | 22.0 |
| 5. Cross-linked dimethylpolysiloxane (note 1) | 15.0 |
| 6. Aluminum zirconium tetrachlorohydrate | 15.0 |
| 7. Perfume | proper |
| (note 1): KSG-16 (trade name, a product of Shin-Etsu Chemical Co., Ltd.) | |

### [Preparation Process]

(A) The ingredients 1 to 5 were mixed homogeneously under heating.
(B) The ingredients 6 and 7 were added to the mixture obtained in the step (A), and dispersed therein.
(C) The dispersion obtained in the step (B) was packed into products.

The solid antiperspirant thus obtained spread smoothly, gave a refreshing feeling, and had neither tacky nor oily feel. Further, it caused no change with temperature variations and a lapse of time. In other words, the antiperspirant according to the invention had excellent usability and stabiliby.

| Example 20: Antiperspirant of Solid Type | |
|---|---|
| Ingredients | Amount mixed (weight %) |
| 1. Silicone-modified wax (II) obtained in Synthesis Example 2 | 26.0 |
| 2. Dimethylpolysiloxane (6 mm²/sec at 25°C) | 22.0 |
| 3. Decamethylcyclopentasiloxane | 22.0 |
| 4. Cross-linked dimethylpolysiloxane (note 1) | 15.0 |
| 5. Aluminum zirconium tetrachlorohydrate (glycine salt) | 15.0 |
| 6. Perfume | proper |
| (note 1) : KSG-16 (trade name, a product of Shin-Etsu Chemical Co., Ltd.) | |

### [Preparation Process]

(A) The ingredients 1 to 4 were mixed homogeneously under heating.
(B) The ingredients 5 and 6 were added to the mixture obtained in the step (A), and dispersed therein.
(C) The dispersion obtained in the step (B) was packed into products.

The thus obtained solid antiperspirant spread smoothly, gave a refreshing feeling, and had neither tacky nor oily feel. Further, it caused no change with temperature variations and a lapse of time. In other words, the antiperspirant according to the invention had excellent usability and stability.

| Example 21: UV-Cut-Off Cream of Water-in-Oil Type | |
|---|---|
| Ingredients | Amount mixed (weight %) |
| 1. Silicone-treated zinc oxide | 20.0 |
| 2. Acrylsilicone resin (note 1) | 12.0 |
| 3. Decamethylcyclopentasiloxane | 20.0 |
| 4. Glyceryl trioctanoate | 3.0 |
| 5. Silicone-modified wax (I) obtained in Synthesis Example 1 | 2.0 |
| 6. Cross-linked polyether-modified silicone (note 2) | 5.0 |
| 7. Polyether-modified silicone (note 3) | 1.0 |
| 8. Alkyl-polyether-comodifited silicone (note 4) | 1.0 |
| 9. Octyl methoxycinnamate | 6.0 |
| 10. Sodium citrate | 0.2 |
| 11. Dipropylene glycol | 3.0 |
| 12. Antiseptic | proper |
| 13. Perfume | proper |
| 14. Purified water | 26.8 |
| (note 1) : KP-545 (trade name, a product of Shin-Etsu Chemical Co., Ltd.) | |
| (note 2) : KSG-21 (trade name, a product of Shin-Etsu Chemical Co., Ltd.) | |
| (note 3) : KF-6017 (trade name, a product of Shin-Etsu Chemical Co., Ltd.) | |
| (note 4): SKF-6026 (trade name, a product of Shin-Etsu Chemical Co., Ltd.) | |

### [Preparation Process]

(A) A portion of the ingredient 3 and the ingredients 4 to 9 were mixed together under heating.
(B) The ingredients 10, 11, 12 and 14 were mixed together, then admixed with the mixture obtained in the step (A) , and further emulsified with stirring.
(C) The ingredients 1 and 2 and the remaining portion of the ingredient 3 were mixed and dispersed, added to the emulsion obtained in the step (B) together with the ingredient 3, and further homogenized.

The thus obtained UV-cut-off cream of water-in-oil type spread easily, and had neither oily nor tacky feel but a fresh feel. Further, it gave a transparent feeling to users' skin and permitted makeup effect to last long. In addition, it caused no change with temperature variations and a lapse of time, and had very excellent usability and stability.

| Example 22: UV-Cut-Off Milky Lotion of Water-in-Oil Type | |
|---|---|
| Ingredients | Amount mixed (weight %) |
| 1. Dimethylpolysiloxane (6 mm²/sec at 25°C) | 5.0 |
| 2. Cross-linked polyether-modified silicone (note 1) | 5.0 |
| 3. Glyceryl trioctanoate | 2.0 |
| 4. Silicone-modified wax (I) obtained in Synthesis Example 1 | 1.0 |
| 5. Polyether-modified silicone (note 2) | 1.0 |
| 6. Titanium dioxide-decamethylcyclopentasiloxane dispersion (note 3) | 30.0 |
| 7. Zinc oxide-decamethylcyclopentasiloxane dispersion (note 4) | 30.0 |
| 8. Dipropylene glycol | 3.0 |
| 9. Sodium citrate | 0.2 |
| 10. Antiseptic | proper |
| 11. Perfume | proper |
| 12. Purified water | 22.8 |
| (note 1): KSG-21 (trade name, a product of Shin-Etsu Chemical Co., Ltd.) | |
| (note 2) : KF-6017 (trade name, a product of Shin-Etsu Chemical | |
| Co., Ltd.) | |
| (note 3) : SPD-T1S (trade name, a product of Shin-Etsu Chemical Co., Ltd.) | |
| (note 4) : SPD-Z1 (trade name, a product of Shin-Etsu Chemical Co., Ltd.) | |

### [Preparation Process]

(A) The ingredients 1 to 5 were mixed together under heating.
(B) The ingredients 8 to 10 and 12 were mixed and dissolved, then admixed with the mixture obtained in the step (A), and further emulsified with stirring.
(C) The ingredients 6, 7 and 11 were added to the emulsion obtained in the step (B) and homogenized.

The thus obtained UV-cut-off milky lotion of water-in-oil type spread easily, and had neither oily nor tacky feel but a fresh feel. Further, it gave a transparent feeling to users' skin and permitted makeup effect to last long. In addition, it caused no change with temperature variations and a lapse of time, and had very excellent usability and stability.

| Example 23: UV-Cut-Off Cream of Oil-in-Water Type | |
|---|---|
| Ingredients | Amount mixed (weight %) |
| 1. Cross-linked organopolysiloxane (note 1) | 5.0 |
| 2. Cetyl isooctanoate | 5.0 |
| 3. Silicone-modified wax (I) obtained in Synthesis Example 1 | 1.0 |
| 4. Titanium dioxide-decamethylcyclopentasiloxane dispersion (note 2) | 15.0 |
| 5. Polyether-modified silicone (note 3) | 1.0 |
| 6. Polyether-modified silicone (note 4) | 1.0 |
| 7. Acrylic acid amide mixture (note-5) | 2.0 |
| 8. Propylene glycol | 5.0 |
| 9. Methyl cellolose (2 % aq. soln.) (note 6) | 5.0 |
| 10. Antiseptic | proper |
| 11. Perfume | proper |
| 12. Purified water | 60.0 |
| (note 1) : KSG-18 (trade name, a product of Shin-Etsu Chemical Co., Ltd.) | |
| (note 2): SPD-T1S (trade name, a product of Shin-Etsu Chemical Co., Ltd.) | |
| (note 3): KF-6027 (trade name, a product of Shin-Etsu Chemical Co., Ltd.) | |
| (note 4): KF-6011 (trade name, a product of Shin-Etsu Chemical Co., Ltd.) | |
| (note 5): SEPIGEL 305 (trade name, a product of SEPIC) | |
| (note 6): Metholose SM-4000 (trade name, a product of Shin-Etsu Chemical Co., Ltd.) | |

### [Preparation Process]

(A) The ingredients 5 to 8, 10 and 12 were mixed together.
(B) The ingredients 1 to 3 were mixed under heating, then admixed with the mixture obtained in the step (A), and further emulsified with stirring.
(C) The ingredients 4 were added to the emulsion obtained in the step (B), further admixed with the ingredients 9 and 11, and homogenized.

The thus obtained UV-cut-off cream of oil-in-water type spread easily, and had neither oily nor tacky feel but a fresh feel. Further, it gave a transparent feeling to users' skin feel. Further, it gave a transparent feeling to users' skin and permitted makeup effect to last long. In addition, it caused no change with temperature variations and a lapse of time, and had very excellent usability and stability.

### Industrial Applicability

The present cosmetic materials spread smoothly and have a fresh feel. Further, they are highly repellent to sweat and water. When applied to the skin or hair, they can impart thereto elasticity, smoothness and emollient effect, and besides, they ensure very natural luster. Moreover, the present cosmetic materials have excellent storage stability. Thus the present cosmetic materials are of great significance from a viewpoint of practical applicability.

## Claims

1. A cosmetic material comprising silicone-modified wax as Component (A)., **characterized in that** the silicone-modified wax is wax in which low-molecular-weight polyethylene and/or low-molecular-weight polypropylene is linked to silicone via an ester linkage.

2. A cosmetic material as described in claim 1, wherein the silicone-modified wax of Component (A) is wax produced by reacting carboxyl group- and/or carboxylic anhydride-containing polyethylene and/or polypropylene wax with a double bond-containing epoxy compound, and thereafter subjecting the reaction product to reaction with a hydrosilyl group-containing silicone.

3. A cosmetic material as described in claim 1 or 2, further comprising unctuous agents as Component (B).

4. A cosmetic material as described in claim 3, wherein at least one of the unctuous agents of Component (B) is an unctuous agent in a liquid state at room temperature.

5. A cosmetic material as described in claim 3, wherein at least one of the unctuous agents of Component (B) is a solid unctuous agent having a melting point of 50°C or higher.

6. A cosmetic material as described in any of claims 3 to 5, wherein at least one of the unctuous agents of Component (B) is a straight-chain or cyclic silicone oil represented by R¹ₐSiO_{(4-a)/2} wherein R¹ is a hydrogen atom or a group selected from 1-30C alkyl groups, aryl groups, aralkyl groups or fluorinated alkyl groups and a is a number satisfying a relation 0≤a≤2.5.

7. A cosmetic material as described in any of claims 3 to 6, wherein at least one of the unctuous agents of Component (B) is a fluorinated or amino group-containing unctuous agent.

8. A cosmetic material as described in any of claims 1 to 7, further comprising water as Component (C).

9. A cosmetic material as described in any of claims 1 to 8, further comprising as Component (D) a compound having an alcoholic hydroxyl group in its molecular structure.

10. A cosmetic material as described in claim 9, wherein the compound of Component (D) having an alcoholic hydroxyl group in its molecular structure is monohydric water-soluble alcohol, polyhydric water-soluble alcohol or a mixture thereof.

11. A cosmetic material as described in any of claims 1 to 10, further comprising as Component (E) a high molecular compound capable of dissolving or swelling in water.

12. A cosmetic material as described in any of claims 1 to 11, further comprising powders, coloring agents or mixtures thereof as Component (F).

13. A cosmetic material as described in claim 12, wherein at least one of the powders, coloring agents and the mixtures thereof as Component (F) is at least one fine powder selected from fine powders of cross-linked silicones having structures formed by cross-linking dimethylsilicones, fine powders of polymethylsilsesquioxanes, hydrophobicity-imparted silica or complex fine powders prepared by coating spherical silicone rubber powder with polymethylsilsesquioxane particles.

14. A cosmetic material as described in claim 12 or 13, wherein at least one of the powders, coloring agents and the mixtures thereof as Component (F) is a fluorinated group-containing powder, a coloring agent or a mixture thereof.

15. A cosmetic material as described in any of claims 1 to 14, further comprising a surfactant as Component (G).

16. A cosmetic material as described in claim 15, wherein the surfactant of Component (G) is a straight-chain or branched silicone containing at least one polyoxyalkylene chain in a molecule.

17. A cosmetic material as described in claim 15 or 16, wherein the surfactant of Component (G) has a HLB value of 2 to 8.

18. A cosmetic material as described in any of claims 1 to 17, further comprising cross-linked organopolysiloxanes as Component (H).

19. A cosmetic material as described in claim 18, wherein the cross-linked organopolysiloxanes are cross-linked organopolysiloxanes which cause swelling when they contain a silicone having low viscosity of from 0.65 to 10.0 mm²/sec at 25°C in a quantity larger than the weight of the cross-linked organopolysiloxanes themselves.

20. A cosmetic material as described in claim 18 or 19, wherein the cross-linked organopolysiloxanes of Component (H) are organopolysiloxanes having a cross-linked structure formed by reaction between the hydrogen atoms bonded directly to silicon atoms and a cross-linking agent having at least two vinylic reactive moities per molecule.

21. A cosmetic material as described in any of claims 18 to 20, wherein the cross-linked organopolysiloxanes of Component (H) are organopolysiloxanes having in their cross-links at least one kind of moiety selected from polyoxyalkylene, alkyl, alkenyl, aryl and fluoroalkyl moieties.

22. A cosmetic material as described in any of claims 1 to 21, further comprising silicone resin as Component (I).

23. A cosmetic material as described in claim 22, wherein the silicone resin of Component (I) is an acrylsilicone resin.

24. A cosmetic material as described in claim 24, wherein the acrylsilicone resin of Component (I) is an acrylsilicone resin containing at least one moiety selected from pyrrolidone, long-chain alkyl, polyoxyalkylene and fluoroalkyl moieties.

25. A cosmetic material as described in claim 22, wherein the silicone resin is at least one silicone compound selected from reticular silicone compounds represented by MQ, MDQ, MT, MDT and MDTQ.

26. A cosmetic material as described in claim 25, wherein the reticular silicone compound is a reticular silicone compound containing at least one moiety selected from pyrrolidone, long-chain alkyl, polyoxyalkylene, fluoroalkyl and amino moieties.

27. A skincare cosmetic material containing a cosmetic material as described in any of claims 1 to 26 as at least one constituent.

28. A makeup cosmetic material containing a cosmetic material as described in any of claims 1 to 26 as at least one constituent.

29. A hair-care cosmetic material containing a cosmetic material as described in any of claims 1 to 26 as at least one constituent.

30. An antiperspirant cosmetic material containing a cosmetic material as described in any of claims 1 to 26 as at least one constituent.

31. An UV protective cosmetic material containing a cosmetic material as described in any of claims 1 to 26 as at least one constituent.

32. A cosmetic material as described in any of claims 1 to 31, which is prepared in a state selected from liquid, emulsion, cream, solid, paste, gel, powder, pressed cake, multilayer, mousse, spray, stick and pencil.

33. A cosmetic material **characterized by** containing 5 to 95 weight % of silicone-modified wax of the aforesaid Component (A) described above and 95 to 5 weight % of unctuous agents of the aforesaid Component (B) and having the form of solid or stick.
